(19) Europäisches Patentamt
European Patent Office
Office européen des brevets

(11) Publication number: 0 231 399 A1

(12) EUROPEAN PATENT APPLICATION

(21) Application number: 85307139.7

(22) Date of filing: 04.10.85

(51) Int. Cl.[4]: C07C 67/38 , C07C 69/52 , C07C 69/34

(43) Date of publication of application: 12.08.87 Bulletin 87/33

(84) Designated Contracting States: DE FR GB NL

(71) Applicant: TEXACO DEVELOPMENT CORPORATION
2000 Westchester Avenue
White Plains New York 10650(US)

(72) Inventor: Lin, Jiang-Jen
2617 Oak Meadow Drive
Round Rock, TX 78664(US)
Inventor: Knifton, John Frederick
10900 Catskill Trail
Austin, TX 78750(US)

(74) Representative: Brock, Peter William et al
Michael Burnside & Partners 2 Serjeants' Inn
Fleet Street
London EC4Y 1HL(GB)

(54) **A process for the production of dicarboxylic acid esters.**

(57) Dicarboxylic acid esters can be produced in good yields and high selectivity by the reaction of alpha olefins or conjugated dienes with carbon monoxide and oxygen at elevated temperature and presure in the presence of a $C_{1-20}$ alkanol and a novel palladium catalyst. The catalyst is a heterogeneous catalyst having palladium supported on a solid substrate and associated with a copper-containing co-catalyst and a lithium-containing co-catalyst.

EP 0 231 399 A1

# A PROCESS FOR THE PRODUCTION OF DICARBOXYLIC ACID ESTERS

This invention relates to the production of dicarboxylic acid esters by the oxidative carbonylation of unsaturated compounds, especially of alpha olefins and conjugated aliphatic dienes. More particularly this invention relates to the use of a novel heterogenous palladium catalyst for the carbonylation process. In this process, dienes, such as 1,3-butadiene, can be converted into hexenedioic acids and their esters, including dimethyl hex-3-ene-1,6-dioate. Adipic acid can then be produced from this hex-3-ene-1,6-dioate ester by successive reduction and hydrolysis. Similarly, alpha olefins such as propylene and 1-octene can be converted into alkyl succinates, e.g. dimethyl methylsuccinate and dimethyl n-hexyl-succinate respectively.

The addition of carbon monoxide to olefins (carbonylation) has long been considered in the art to be a highly attractive route to a number of commercially valuable chemical products. It is known in the art to prepare unsaturated aliphatic carboxylic acids and their esters by the catalytic oxidative carbonylation of alpha olefins and dienes. More particularly, it is known to synthesize aliphatic carboxylic acids and their esters by reactive carbon monoxide, oxygen and an olefin such as propylene or 1-octene, or a conjugated aliphatic diene such as 1,3-butadiene, isoprene, 2,3-dimethylbutadiene, or 1,3-pentadiene, at elevated temperature and pressure, in the presence of various catalysts, often in the presence of dehydrating agents. One useful diester is dimethyl hex-3-ene-1,6-dioate, which is a precursor for adipic acid. Adipic acid is a large volume chemical used, for example, in making nylon-66 polymer for fibres and plastics, as well as in polyurethane foams. Another useful type of ester is an alkyl succinate, which is a precursor for alkyl succinic anhydrides or diols. The related polyalkenyl succinic anhydride is used for lubricating oil additives and for polyester resins.

Some of the early patents in the field, such as US-A-3397226, -3397225, -3481845 and -3755421, demonstrate the use of catalysts comprising a platinum group metal salt or chelate, and a salt of a multivalent heavy metal which functions as a redox agent, for the oxidative carbonylation of olefins to produce esters of unsaturated carboxylic acids, esters of dicarboxylic acids and esters of beta-alkoxy-substituted carboxylic acids.

In US-A-4281173, there is disclosed a process for the preparation of unsaturated diesters in the presence of a catalytic amount of a platinum group metal compound, a copper or iron oxidant salt compound, a soluble vanadium salt or a stoichiometric amount of a dehydrating agent. An anhydrous halogen-containing acid may optionally be included. Butadiene is the starting substrate in this patent, but the examples disclose only the use of a homogeneous palladium catalyst system, the effect of supported palladium systems remains unknown.

WO-80/00250 relates to the carbonylation of conjugated dienes such as 1,3-butadiene, by the addition of carbon monoxide and an alcohol of the formula ROH, in the presence of a palladium (II) salt, a copper - (II) salt and a base. This synthesis requires the use of stoichiometric quantities of copper (II) salt, but does not require oxygen.

J. Org. Chem. 1979, 44(20), 3474-82, discloses the methoxycarbonylation of a variety of olefins with methanol and carbon monoxide in the presence of palladium, using stoichiometric amounts of copper (II) chloride as reoxidant, and sodium butyrate as buffer. Different aliphatic carboxylic acid diesters were formed in varying yields, depending on the choice of olefin and the carbon monoxide pressure. The reaction usually resulted in the addition of two carbomethoxy groups to the double bond.

JP-A-828,942 discloses the preparation of diesters by the addition of carbon monoxide, oxygen and alcohols to conjugated dienes in the presence of $PdCl_2$ and $CuCl_2$ along with $BuNH_2$ in dioxan.

J. Am. Chem. Soc. 98, 1810 (1976), provides much data on the yields of various esters using different cyclic and acyclic olefins. The effects of some of the cocatalysts used in these reactions, yields of products, and effect of added base were also discussed. Palladium (II) chloride was the catalyst, and a stoichiometric amount of copper (II) chloride was used as reoxidant.

J. Org. Chem. 37 2034 (1972) demonstrates that, in a palladium redox system, optimum results are achieved by restricting excess amounts of both hydrogen and chloride ions.

US-A-4230881 discloses a binary system for preparation of organic esters, such as dimethyl oxalate, in which the principal member is a palladium complex and the cocatalyst is preferably an organic compound having an acidic nature, no matter how weak. With this system, esters are prepared without employing any oxygen and without formation of water..

US-A-4269781 discloses reacting 1,3-butadiene with carbon monoxide and an alkanol containing 0.5 to 10 weight % of water, in the presence of a catalyst complex of palladium and tertiary phosphine ligand, to yield a liquid phase product mixture containing alkyl nonadienoate; contacting the product mixture with a hydrocarbon solvent to form two liquid phases, separating the two liquid phases, and recovering alkyl nonasienoate from the hydrocarbon solvent phase.

US-A-4281174 discloses preparing dialkyl oxalates by reacting CO and air with an alcohol in the presence of a catalyst comprising palladium in complex combination with a ligand, a small amount of quinone, and a redox agent. The quinone component helps improve yields.

J. Mol. Cat. 18 (1983) 109-112 describes the dimerization-monocarbonylation of butadiene, the catalytic activity of a $Pd(OAC)_2/R_3P$ catalyst system being improved by adding maleic anhydride. It was suggested therein that the maleic anhydride stabilizes the Pd(O) species through coordination after the catalytic cycle is completed.

In many processes known in the art, separation of the high boiling aliphatic carboxylic acid or ester product from the catalyst system can be difficult. It would be advantageous to devise a catalyst system which heterogenous, which improves the product distribution to desired carboxylic acid, and which improves the ease of product/catalyst separation. A supported (palladium-containing) catalyst system which allowed for easier separation of product from catalyst by filtration would be more efficient and far more attractive commercially. Furthermore, a suitable support for such a palladium catalyst system may be selected so as to improve both the productivity of desired carboxylic acid/ester derivative and the selectivity to said desired product or products.

It would be extremely advantageous if such a system produced a higher yield of linear aliphatic carboxylic acid ester precursors of adipic acid.

The present invention provides a process for the production of dicarboxylic acid esters by the reaction of alpha-olefins or conjugated dienes with carbon monoxide and oxygen at elevated temperature and pressure in the presence of a $C_{1-20}$ alkanol and a palladium catalyst, in which the palladium catalyst is a heterogeneous catalyst having palladium supported on a solid substrate and associated with a copper-containing co-catalyst and a lithium-containing co-catalyst.

According to one embodiment of the invention, the diene is a conjugated diolefin having 4 to 12 carbon atoms and containing a group of the formula:

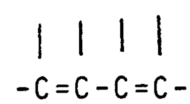

wherein each carbon is bonded either to hydrogen or a hydrocarbyl group, whereby to form an aliphatic dicarboxylic acid ester containing a group of the formula:

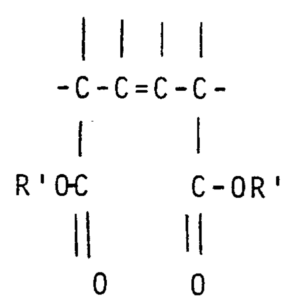

wherein R' is $C_{1-20}$ alkyl.

According to another embodiment of the invention, the alpha-olefin has the formula:

$R\text{-}CH=CH_2$

wherein R is hydrogen or $C_{1-10}$alkyl, whereby to form an aliphatic dicarboxylic acid ester having the formula:

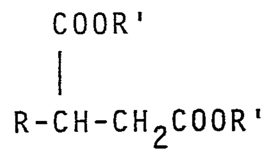

wherein R is hydrogen or $C_{1-10}$ alkyl, and R' is $C_{1-20}$alkyl.

By the process of the invention, butadiene can be converted into adipic acid precursors, including dialkyl hexenedioates, and by-products, including alkyl 5-methoxy-3-pentenoate, dialkyl carbonate, dialkyl oxalate and 4-vinyl-cyclohexene.

This invention demonstrates improved linearity of product (in the case of 1,4-addition to dienes), improved ratio of desired to undesired products, and improvement in ease, efficiency and commercial attractiveness of means of separation of product.

In accordance with one embodiment of the invention, aliphatic dienes having a conjugated diene moiety of the formula:

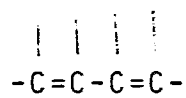

wherein each carbon is bonded to hydrogen or a hydrocarbyl group, are converted, by 1,4-addition of carbon monoxide, oxygen and alkanol, into aliphatic, unsaturated dicarboxylic esters in which the conjugated double bonds have been transformed into a moiety having the formula:

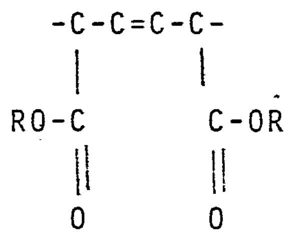

wherein R is an alkyl group. The process comprises passing the aliphatic conjugated diolefin, carbon monoxide and oxygen together with alkanol over the heterogenous supported palladium catalyst in the presence of the copper and lithium cocatalysts. The reactants and catalyst components are charged to a reaction vessel and, in the absence of water, subjected to a carbon monoxide pressure and temperature for a sufficient time to effect the desired carbonylation reaction. In the specific use of the diolefin 1,3-butadiene, the carbonylation reaction can be represented by the following equation:

$CH_2=CHCH=CH_2$ + $CO$ + $O_2$ + $CH_3OH$ $CH_3OOCCH_2CH=CHCH_2COOCH_3$ + $CH_3OCH_2CH=CHCH_2COOCH_3$ and

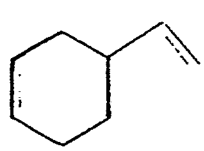 + 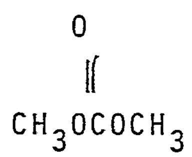 + 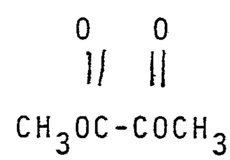

Generally the reaction between the aliphatic conjugated diolefin, carbon monoxide, oxygen and alkanol may be carried out in an autoclave or other appropriate reactor. Although the order of addition of reactants and catalyst components may vary, a general procedure is to charge the supported palladium catalyst, copper-containing cocatalyst, lithium-containing cocatalyst, aliphatic conjugated diolefin, alkanol and optional dehydrating agent to an appropriate reactor, such as a stainless-steel, reactor, then to introduce the proper amount of carbon monoxide and oxygen and increase the pressure and temperature to a desired level for an appropriate period to produce the desired aliphatic carboxylic acid derivative.

Diolefins suitable for use in the present invention are aliphatic conjugated diolefins having 4 to 12 carbon atoms per molecule with a bond system of the general formula:

$$-C=C-C=C-$$

wherein each carbon is bonded to hydrogen or a hydrocarbyl group.

Suitable aliphatic conjugated dienes include 1,3-butadiene, piperylene, 1,3-hexadiene, isoprene, 2,4-hexadiene, 2-methyl-1,3-pentadiene, 3-methyl-1,3-pentadiene, 1,3-cyclohexadiene, 2,4-dimethyl-1,3-pentadiene, 1,3-cyclooctadiene, 2,5-dimethyl-2,4-hexadiene and 2,3-dimethyl-2,4-hexadiene. The preferred aliphatic conjugated diene for the practice of this invention is 1,3-butadiene.

In another embodiment, the preferred alpha-olefins have the formula:

$R\text{-}CH=CH_2$

wherein R is an alkyl or hydrogen and are converted, by 1,2-addition into aliphatic, dicarboxylic esters having the formula:

$$R\text{-}CH(COOR')\text{-}CH_2\text{-}COOR'$$

wherein R' is $C_{1\text{-}20}$ alkyl group derived from the alkanol, such as a methyl group from methanol. The process can generally be conducted as described above in the case of the dienes. In the specific instance where the olefin is 1-octene, the carbonylation reaction can be represented by the following equation:

$$CH_3(CH_2)_5CH=CH_2 + CH_3OH + CO/O_2 \quad CH_3(CH_2)_5CH(COOCH_3)\text{-}CH_2\text{-}COOCH_3$$

when propylene is used, the reaction can be represented by the following equation:

$$CH_3CH=CH_2 + CH_3OH + CO/O_2 \quad CH_3CH(COOCH_3)\text{-}CH_2\text{-}COOCH_3$$

Olefins suitable for use in the present invention are simple olefins containing 2 to 12 carbon atoms per molecule and have the general formula:

$R\text{-}CH=CH_2$

wherein R is hydrogen or $C_{1\text{-}10}$ alkyl.

Suitable alpha-olefins include ethylene, propylene, 1-butene, 1-pentene, 1-hexene, 1-heptene, 1-octene, 1-nonene, 1-decene, 1-undecene, and 1-dodecene. The preferred $\alpha$-olefins are 1-octene and propylene.

Suitable $C_{1\text{-}20}$ alkanols for use in accordance with the invention include methanol, ethanol, n-propanol, iso-propanol, tert-butanol, n-butanol, n-hexanol, n-decanol and n-dodecanol. The preferred alkanol is methanol.

As mentioned herinabove, a palladium-containing catalyst is employed in the process of the invention, and together with the particular cocatalysts provides a catalyst system which demonstrates an increase in selectivity to linear carboxylic acid ester products and which allows for ease of separation, a feature which is commercially attractive and desired in the art. The palladium is supported on an inert substrate such as alumina, silica-alumina, silica gel, kaolin, keiselguhr, zirconium oxide, titania, barium carbonate, or silicalite, as well as certain zeolitic silica-aluminas such as 4A-molecular sieve, and certain activated carbons. The preferred palladium-containing catalyst is palladium-on-active carbon. In this instance, the palladium concentration on the activated carbon support may, for instance, be from 0.1 to 20 wt %, and may be higher, but this is the range normally employed, with the preferred range being from 0.5 to 5.0 wt %.

The support may be in the form of powders, pellets, spheres, shapes and extrudes. They should also be of suitable porosity, such that they may be employed in fixed or fluidized beds. In the process of this invention palladium on graphite (1 wt %) was found to be the preferred form of the catalyst. Based on converted butadiene, the adipic acid precursor hex-3-ene-1, 6 dioate was produced at 85% selectivity. Similarly dimethyl n-hexyl-succinate was produced at 94% selectivity and 61% conversion of 1-octene.

The palladium-containing precursor compound to be dispersed upon the solid substrate may be impregnated on the substrate in the form of a salt of bivalent palladium possibly as the salt of a carboxylic acid, such as palladium acetate, palladium propionate, or as other organic or inorganic salts, e.g. palladium acetylacetonate or palladium nitrate. Alternatively it can be added in the form of a palladium halide, such as palladium (II) chloride.

Generally, said palladium-containing catalyst is prepared by first dissolving or slurrying the selected palladium salt, e.g. palladium (II) chloride, with a suitable solvent system and subsequently impregnating the selected inert support or carrier (substrate) with the palladium-containing mixture. The solutions or slurries may be poured onto the carrier, or the solid carrier may be immersed in an excess of the liquid solution or slurry with any excess being subsequently removed.

The impregnated support is then maintained at a temperature sufficient to volatize the solvent component, e.g. at a temperature between 100 and 500°C, to permit drying of the composite solid catalyst. Reduced pressure may also be applied to the catalyst in order to volatize the solvent, although this is not essential. During this stage of the process, the volatile solvent evaporates from the solid catalyst, and the palladium component remains on the support.

The solvent which may be used to dissolve the palladium compound before impregnation of the support should be a liquid of relatively low boiling point, for example, 150°C or less. A preferable group of solvents include mineral acid solutions such as hydrochloric acid and nitric acid, carboxylic acids such as acetic acid and propionic acid, halogenated solvents such as chloroform and carbon tetrachloride, ketones such as acetone and methyl isobutyl ketone, alcohols such as methanol, iso-propanol and tert-butanol, aromatics such as benzene, toluene and xylene, and certain heterocyclic solvents, such as pyridine and N-methyl-pyrrolidone. The choice of solvent is dependent upon the nature of the palladium-containing compound to be used for impregnation.

In accordance with this invention a copper compound is used as a cocatalyst. The copper-containing cocatalyst can be added to the reactor in the form of a salt of copper, such as a halide, sulfate, trifluoroacetate, nitrate, naphthoate, hex-3-endioate or acetate. Copper salts which work include, but are not limited to, copper (II) chloride, copper (II) bromide, copper (II) sulfate, copper (II) trifluoroacetate, copper (II) acetate, copper (II) triflate, copper (II) fluorosulfonate, copper (I) chloride and copper (I) sulfate. The preferred compound is copper (II) chloride.

A lithium-containing cocatalyst is also used in addition to the copper-containing cocatalyst. The lithium compound is, for instance, a salt, such as lithium halide, sulfate, nitrate and acetate. Specific examples include lithium chloride, lithium bromide, lithium iodide and lithium acetate. The preferred lithium compound is lithium chloride. Optionally a dehydrating agent may also be added to the reaction mixture. Suitable dehydrating agents include acetals and ketals. These may include acetaldehyde dimethyl acetal, benzal dehyde dimethyl acetal and formaldehyde dimethyl acetal. Suitable ketals can be 2,2-dimethoxypropane, and dimethoxymethane. The dehydrating agent may be used in a wide range of ratios compared with the quantity of aliphatic alpha-olefin or diene charge, but preferably 1 to 2 mols, or even more, of dehydrating agent, such as 2,2-dimethoxypropane, are employed per mol of alpha-olefin or diene.

The process of the present invention can be suitably performed by introducing the oxygen, carbon monoxide and alcohol at a desired pressure into contact with the alpha-olefin or diene, preferably 1-octene, propylene or butadiene, optional dehydrating agent, the supported palladium catalyst, the copper-containing cocatalyst and the lithium-containing cocatalyst, and heating to the desired temperature.

In general a carbon monoxide pressure of 0.4 to 35 MPa partial pressure, and preferably 3.5 to 12.5 MPa is employed. A least stoichiometric quantities of carbon monoxide are generally employed. An excess of carbon monoxide may be employed however, particularly in continuous processses. Where a large amount of carbon monoxide is employed, the unreacted carbon carbon monoxide may be recycled.

The partial pressure of oxygen is generally selected so that the mol ratio of carbon monoxide to oxygen is from 1:1 to 100:1. A carbon monoxide to oxygen mol ratio of 5:1 to 20:1 has been employed in this work for the synthesis of hexenedioic acid esters from 1,3-butadiene, and of succinate esters from the alpha-olefins and is considered to be the preferred range.

The reaction will proceed at temperatures above 25°C. It is generally preferred to operate the process at a temperature from 80 to 150°C, to obtain a convenient rate of reaction with the particular olefin or diene.

The reaction time is generally dependent upon the olefin or diene being reacted, the temperature and pressure, and the amounts and type of catalyst, cocatalyst and dehydrating agent being employed. Reaction time will vary dependent on whether the process is continuous or batch wise and may be from 1 to 15 hours. Reaction time is generally about 2 hours.

The quantity of palladium catalyst, and copper lithium cocatalyst employed in the instant invention is not critical, and may vary over a wide range. In general, the carbonylation process is desirably conducted in the presence of a catalytically effective quantity of the active palladium species which gives the desired ester products in reasonable yields.

Concentrations of copper compound may be between 0.1 and 50 wt %, with the preferred range being 0.1 to 1 wt %, and optimally about 0.5 wt %. Higher concentrations of copper-containing co-catalyst may be used, e.g. up to 50 wt %.

Concentrations of lithium compound may be between 0.0001 and 1.0 wt %, with the preferred range being 0.001 to 0.1 wt %, and optimally about 0.005 wt %. Higher concentrations of lithium compound may be used, e.g. up to 1.0 wt %.

The ratio of supported palladium compound to copper-containing cocatalyst to lithium-containing cocatalyst is not critical. Good results are obtained using a weight ratio Pd:Cu:Li of about 0.01:1.0:0.005.

In reacting the alpha-olefin or diene, carbon monoxide, oxygen and alkanol in the presence of the catalyst to form and alkyl succinate or a hexenedioic acid diester, whether continuously or batch wise, the carbon monoxide may also be used in conjunction with up to 50% by volume of one or more other gases. These other gases may include one or more inert gases, such as nitrogen, argon or neon, or they may include gases that may or may not undergo reaction under CO carbonylation conditions such as carbon dioxide, hydrogen, hydrocarbons such as methane, ethane, and propane, ethers such as dimethyl ether, methylethyl ether and diethyl ether and esters such as methyl acetate.

The 1,3-butadiene carbonylation process disclosed herein leads to the formation of two classes of product. The primary product is the hexenedioic acid diester, dimethyl hex-3-ene-1,6-dioate, which can be reduced and hydrolyzed to adipic acid. By-products include 5-methoxy-3-pentenoate, dimethyl carbonate and dimethyl oxalate.

The 1-olefin carbonylation process also leads to the formation of two classes of product. The primary product is the dialkyl ester, e.g. dimethyl n-hexyl-succinate (from 1-octene) or dimethyl methyl-succinate (from propylene). By-products include dimethyl carbonate and beta-alkoxy-carboxylates.

The benefits of the improved oxidative dicarbonylation process, using the supported palladium catalyst, copper-containing cocatalyst, lithium-containing cocatalyst and optional dehydrating agent are:

a) increased productivity and selectivity to desired ester product, and

b) ease of separation of said ester from the catalyst.

The process of this invention can be conducted in a batch wise semi-continuous or continuous fashion. The solid catalyst may be employed as a fixed bed. The reactor may consist of a series of catalyst beds or the catalyst may be placed in tubes with a heat exchange medium around the tubes. In order to provide certain operating advantages, the metal content of the catalyst may be varied through the reactor bed, and the reactants may be passed in upwards or downwards flow through the reactor.

To ensure maximum yields of desirable products, contact between the liquid reaction mixture and any iron-rich metal surfaces should be limited wherever possible during the carbonylation step. One means by which this contact can be minimized is by carrying out the reaction in a glass-lined reactor. An alternative method is to use the carbonylation reactor lined with some other inert material such as a silver-lined reactor. Further alternatives include the use of titanium-lined pressure reactors, tantalum-lined reactors, and reactors having Hastelloy ® alloy or copper nickel alloy surfaces.

Generally, operating conditions can be adjusted to optimize the formation of any desired diester product, and said materials may be recovered by methods well known in the art, such as filtration, distillation, fractionation and extraction.

The products of this improved catalyst system have been identified by one or more of the following analytical procedures, viz, gas-liquid phase chromatography (glc), infrared (ir) mass spectrometry, nuclear magnetic resonance (nmr) and elemental analysis, or a combination of these techniques. All temperatures are in degrees centigrade and all pressures in megapascals (MPa).

The following illustrative examples are submitted to supply specific and illustrative embodiments.

The following equation describes the basic reaction described in Examples 1 to 10. The various products are labeled with Roman numerals for reference.

$CH_2=CHCH=CH_2$ + $CO$ + $O_2$ + $CH_3OH$ catalyst

$CH_3OOCCH_2CH=CHCH_2COOCH_3$ + $CH_3OCH_2CH=CHCH_2COOCH_3$ +

(I) (II)

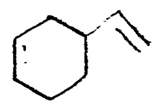 + $CH_3OCOCH_3$ + $CH_3OC{-}COCH_3$

(O above the central carbon of IV; O O above the two carbonyl carbons of V)

(III) (IV) (V)

EXAMPLE 1

A 300 ml stainless-steel reactor was charged with palladium (1.0 wt%) on graphite (1.0 g), copper (II) chloride hydrate (1.2 g) lithium chloride (0.084 g), methanol (0.96 g) and 2,2-dimethoxypropane (20 g). The autoclave was sealed and then charged with 20.0 g of 1,3-butadiene and pressurised with CO (3.54 MPa) and $O_2$ (0.79MPa). The system was heated to 100°C and pressure was raised to 10.44MPa with CO. These conditions were maintained for 2 hours. During the reaction process, the pressure dropped to 8.72 MPa. The reactor was cooled to room temperature and an off-gas sample was taken. The excess gas was vented, and a brown liquid product with solid catalysts at the bottom was recovered (33.3 g).

The glc analysis of liquid products and off-gas samples indicated the following product selectivities.
dimethyl hex-3-ene-1,6-dioate (I) 28%
methyl 5-methoxy-3-pentenoate (II) 5%
4-vinyl-1-cyclohexene (III) 22%
dimethyl carbonate (IV) 31%
dimethyl oxalate (V) 13%
Estimated selectivity to dimethyl hex-3-ene-1,6-dioate (basis butadiene carbonylated) is 85%.

The off-gas analysis showed:
carbon monoxide 92.6%
carbon dioxide 1.1%
total heavies material 5.2%

Examples 2 to 10 were carried out using the same procedure as described in Example 1. Results are shown in the Table. The copper-containing and lithium-containing cocatalysts were the same throughout as that used in Example 1, except for Example 5 where no lithium cocatalyst was employed. Examples 2,3 and 4 use supports other than graphite for the palladium.

TABLE

| Example | Pd-Catalyst | Copper Cocatalyst | LiCl Co-catalyst | MeOH | 2,2-di-methoxy-propane | Reaction conditions | Temp. Time | Wt. gain | Product Selectivities I | II | III | IV | V |
|---|---|---|---|---|---|---|---|---|---|---|---|---|---|
| 2 | 0.5% Pd on Kaolin (2.0 g) | $CuCl_2 \cdot 2H_2O$ (1.20 g) | 0.084 g | 0.96 g | 20 g[a] | 10.44 MPa $CO/O_2$=7 | 100°C 2 h | – | 3 | 1 | 14 | 72 | 7 |
| 3 | " | " | " | " | 20 g[a] | 10.44 MPa $CO/O_2$=7 | " | 0 | – | – | 13 | 84 | – |
| 4 | 0.5% Pd on Al (2.0g) | " | " | " | 20 g[a] | 10.44 MPa $CO/O_2$=15 | 100°C 5 h | 3.6 g | – | – | – | 81 | – |
| 5 * | 1% Pd on graphite (1.0 g) | " | 0 | " | 20 g[a] | 10.44 MPa $CO/O_2$=15 | 100°C 2 h | 4.1 g | 15 | 5 | 16 | 76 | 3 |
| 6 | " | " | 0.084 g | 10 g | 0[a] | 10.44 MPa $CO/O_2$=15 | " | 3.0 g | 26 | 19 | 33 | 8 | 1 |
| 7 | " | " | " | 0.96 g | 20 g[a] | 13.89 MPa $CO/O_2$=20 | " | 2.6 g | 21 | 3 | 15 | 42 | 10 |
| 8 | " | " | " | " | 20 g[b] | 6.13 MPa $CO/O_2$=8 | " | 5.8 g | 8 | ~0 | 30 | 61 | ~0 |
| 9 | " | " | " | " | 20 g[b] | 5.78 MPa $CO/O_2$=8 | 80°C | 2.2 g | 9 | 2 | 15 | 75 | 0 |
| 10 | " | " | " | " | 20 g[b] | 13.89 MPa $CO/O_2$=20 | 80°C 2 h | 0.5 g | 2 | 0 | 14 | 75 | 1 |

(a) Butadiene used (20 g), except (b) 40 g

* For Comparison

It may be noted from a consideration of the data in the Table that:

1) In Examples 1 to 4, showing the use of various palladium catalyst components on different supports, the palladium-on-graphite catalyst gave a significant improvement in selectivity to desired product, dimethyl hex-3-ene-1,6-dioate (I) (Example 1).

2) Production of desired dimethyl hex-3-ene-1,6-dioate (I) is lower, and selectivity to less desirable dimethyl carbonate is higher, when no lithium cocatalyst is used (see Example 5).

3) The ratio of desirable dimethyl hex-3-ene-1,6-dioate (I) to less desirable methyl 5-methoxy-3-pentenoate (II) is lower in the absence of the dehydrating agent, 2,2-dimethoxypropane (see Example 6).

4) Productivity of desired adipic acid precursor(I) is lower when less oxygen is used (see Example 7).

5) Productivity of desired hexenedioic acid ester(I) is lower when less carbon monoxide is used, and the weight ratio of I/IV+V is also lower (see Example 8).

6) Lower operating temperatures (Examples 9 and 10) lead to lower weight gains of liquid products.

EXAMPLE 11

A 300 ml stainless-steel reactor was charged with palladium (1.0 wt%) on graphite (0.50 g), cuprous chloride hydrate (0.60 g), lithium chloride (0.042 g), methanol (0.96 g) and 2,2-dimethoxypropane (20 g). The autoclave was sealed, 10.0 g of propylene was injected followed by pressurising with CO (3.54 MPa) and $O_2$ (0.79 MPa). The system was heated to 100°C and pressure was raised to 13.89 MPa with CO. These conditions were maintained for 2 hours. During the reaction process, the pressure dropped to 11.82 MPa. The reactor was cooled to room temperature and an off-gas sample was taken. The excess gas was vented and a brown liquid product with solid catalysts at the bottom was recovered (23.0 g).

The glc analysis of liquid products and off-gas samples indicated the following product selectivities:

dimethyl alpha-methyl succinate 64%
dimethyl carbonate 32%
unknown 4%
The off-gas analysis showed:
carbon monoxide 77.3%
carbon dioxide .21%
total heavies material 17.5%

EXAMPLE 12

The procedures of Example 11 were repeated except that 1-octene was used as the olefin
dimethyl n-hexyl-succinate 66%
dimethyl carbonate 29%
unknown 5%

Dimethyl carbonate was derived from methanol and carbon monoxide. Dimethyl n-hexyl-succinate was produced from 1-octene, carbon monoxide and methanol. Based on the 1-octene reaction, there was 94% selecrivity to dimethyl n-hexyl-succinate and 61% 1-octene conversion Dimethyl carbonate is a valuable by-product used as a gasoline extender. It is also worthwhile to note that carbon dioxide in the off-gas sample was only 0.127% The off-gas sample showed:
carbon monoxide 93.7%
carbon dioxide 0.127%
oxygen 0.26%
heavy materials 2.6%

EXAMPLE 13

The experimental procedures were repeated, except for using $PdCl_2$ (0.177 g), $CuCl_2$ $H_2$ O (1.2 g), LiCl - (0.084 g), methanol (0.96 g), 2,2-dimethoxypropane (20 g) and 10.0 g of propylene. The liquid products - (33.7 g) showed:
80% selectivity to dimethyl alpha-methylsuccinate,
10% selectivity to dimethyl carbonate, and
10% of unknown materials

The off-gas analysis showed:
90.7% carbon monoxide,
0.19% carbon dioxide,
6.8% heavy materials.

It should be noted that the ratio of dimethyl alpha-methyl-succinate to unknown (regardless of the dimethyl carbonate formation) is different from that obtained in palladium/graphite reactions (Example 11).

EXAMPLE 14

The experimental procedure of Examples 11 to 13 was repeated using $PdCl_2$ (0.177 g), $CuCl_2 XH_2 O$ - (1.2 g), LiCl (0.084 g), methanol (20 g), propylene (10.0 g) and no 2,2-dimethoxypropane.

The reaction gave 19.5 g of liquid product, with the product selectivity, 89% to dimethyl alpha-methyl-succinate and 0% dimethyl carbonate.

EXAMPLE 15 (For Comparison)

Example 13 was repeated using the same catalyst composition and reactants, except that no oxygen was added. The recovered liquid components showed no products (dimethyl alpha-methylsuccinate or dimethyl carbonate). The presence of oxygen was essential for the process of the invention.

**Claims**

1. A process for the production of dicarboxylic acid esters by the reaction of alpha olefins or conjugated dienes with carbon monoxide and oxygen at elevated temperature and pressure in the presence of a $C_{1-20}$ alkanol and a palladium catalyst, characterized in that the palladium catalyst is a heterogeneous catalyst having palladium supported on a solid substrate and associated with a copper-containing co-catalyst and a lithium-containing co-catalyst.

2. A process according to claim 1, characterized in that the diene is a conjugated diolefin having 4 to 12 carbon atoms and containing a group of the formula:

```
 |   |   |   |
-C = C - C = C-
```

wherein each carbon is bonded either to hydrogen or a hydrocarbyl group, whereby to form an aliphatic dicarboxylic acid ester containing a group of the formula:

```
       |   |   |   |
      -C - C = C - C-
       |           |
 R´O - C           C - OR´
       ||          ||
       O           O
```

wherein R´ is $C_{1-20}$ alkyl.

3. A process according to claim 2, characterized in that the conjugated diolefin is 1,3-butadiene.

4. A process according to claim 3, characterized in that the alkanol is methanol, and the resulting dimethyl hex-3-ene-1,6-dioate product is reduced and hydrolysed to adipic acid.

5. A process according to claim 1, characterized in that the alpha olefin has the formula:
$R-CH=CH_2$
wherein R is hydrogen or $C_{1-10}$ alkyl, whereby to form an aliphatic dicarboxylic acid ester having the formula:

$$R-\underset{\displaystyle |}{\overset{\displaystyle COOR'}{CH}}-CH_2COOR'$$

wherein R is hydrogen or $C_{1-10}$ alkyl and R' is $C_{1-20}$alkyl.

6. A process according to claim 5, characterized in that the alpha olefin is propylene or 1-octene.

7. A process according to any preceding claim characterized in that the reaction is carried out in the presence of a liquid drying agent selected from acetals and ketals.

8. A process according to any preceding claim characterized in that the temperature is from 80°C to 150°C, and the pressure is from 3.5 to 12.5 MPa.

9. A process according to any preceding claim characterized in that the copper-containing co-catalyst is cupric chloride, cupric bromide, cupric iodide or cuprous chloride hydrate.

10. A process according to any preceding claim characterized in that the lithium-containing co-catalyst is lithium chloride.

11. A process according to any preceding claim characterized in that the substrate for the heterogeneous palladium catalyst is graphite.

European Patent Office

# EUROPEAN SEARCH REPORT

Application number

EP 85 30 7139

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A-2 064 353 (ATLANTIC RICHFIELD)<br>* Page 1, lines 41-56; page 2, lines 6-23; page 4, lines 13-36; page 6, lines 35-57; page 20, claims 1,9 * & US - A - 4 281 173 (Cat. D) | 1-3,7-10 | C 07 C 67/38<br>C 07 C 69/52<br>C 07 C 69/34 |
| X | GB-A-2 000 495 (ATLANTIC RICHFIELD)<br>* Page 1, line 30 - page 2, line 17; page 2, lines 33-65; page 4, lines 12-33; pages 11-12, claims 1-4,7,15,19,26 * | 1-4,7-10 | |
| X | EP-A-0 070 200 (MONTEDISON)<br>* Page 7 * | 1,5 | |
| | | | TECHNICAL FIELDS SEARCHED (Int. Cl.4) |
| X | DE-A-2 512 062 (TEJIN LTD.)<br>* Page 6; page 7, paragraph 1; page 8, paragraph 2; page 9, paragraph 2; page 11, last paragraph - page 2, paragraph 2; page 14, paragraph 4; page 16, example 15; page 26, claims 1,3,4 * | 1,5-11 | C 07 C 69/00<br>C 07 C 67/00 |

The present search report has been drawn up for all claims

| Place of search | Date of completion of the search | Examiner |
|---|---|---|
| THE HAGUE | 23-05-1986 | KINZINGER J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82